# NEUE EUROPÄISCHE PATENTSCHRIFT

(11) **EP 1 000 167 B2**
(45) Veröffentlichungstag und Bekanntmachung des Hinweises auf die Entscheidung über den Einspruch: **20.04.2011**
(45) Hinweis auf die Patenterteilung: 21.12.2005
(21) Anmeldenummer: 98942591.3
(22) Anmeldetag: 22.07.1998
(51) Int. Cl.: C12N 15/90, C12N 15/10, A61K 38/49, C12N 9/72

(54) **HERSTELLUNG HUMANER MUTIERTER PROTEINE IN HUMANEN ZELLEN MITTELS HOMOLOGER REKOMBINATION**
PRODUCTION OF HUMAN MUTANT PROTEINS IN HUMAN CELLS BY HOMOLOGOUS RECOMBINATION
PRODUCTION DE PROTEINES MUTANTES HUMAINES DANS DES CELLULES HUMAINES PAR RECOMBINAISON HOMOLOGUE

(30) Priorität: 23.07.1997 EP 97112639
(43) Veröffentlichungstag der Anmeldung: 17.05.2000
(73) Patentinhaber: Roche Diagnostics GmbH, 68305 Mannheim (DE)
(72) Erfinder: STERN, Anne, D-82377 Penzberg (DE); HONOLD, Konrad, D-82377 Penzberg (DE)
(74) Vertreter: Weiss, Wolfgang
(86) Internationale Anmeldenummer: PCT/EP1998/004583
(87) Internationale Veröffentlichungsnummer: WO 1999/005264

(56) Entgegenhaltungen:
- EP-A- 0 382 174
- EP-A- 0 386 766
- WO-A-92/03917
- WO-A-92/20808
- WO-A-93/22443
- WO-A-94/04032
- WO-A-95/31560
- WO-A-96/40271
- O. SMITHIES ET AL.: "Insertion of DNA sequences into the human chromosomal beta-globin locus by homologous recombination." NATURE, Bd. 317, 19. September 1985, Seiten 230-234, XP002052217
- T.M. DECHIARA ET AL.: "A growth-deficiency phenotype in heterozygous mice carrying an insulin-like growth factor II gene disrupted by targeting." NATURE, Bd. 345, 3. Mai 1990, Seiten 78-80, XP002052218
- ZIMMER A ET AL: "PRODUCTION OF CHIMAERIC MICE CONTAINING EMBRYONIC STEM (ES) CELLS CARRYING A HOMOEOBOX HOX 1.1 ALLELE MUTATED BY HOMOLOGOUS RECOMBINATION" NATURE, Bd. 338, 9. März 1989, Seiten 150-153, XP002025689
- HASTY P ET AL: "INTRODUCTION OF A SUBTLE MUTATION INTO THE HOX-2.6 LOCUS IN EMBRYONIC STEM CELLS" NATURE, Bd. 350, 21. März 1991, Seiten 243-246, XP002030589
- A.L. JOYNER ET AL.: "Production of a mutation in mouse En-2 gene by homologous recombination in embryonic stem cells." NATURE, Bd. 388, 9. März 1989, Seiten 153-156, XP002052219
- S.L. MANSOUR ET AL.: "Disruption of the proto-oncogene int-2 in mouse embryo-derived stem cells: a general strategy for targeting mutations to non-selectable genes." NATURE, Bd. 336, 24. November 1988, Seiten 348-352, XP002052220
- K.R. THOMAS ET AL.: "Site-directed mutagenesis by gene targeting in mouse embryo-derived stem cells." CELL, Bd. 51, 6. November 1987, Seiten 503-512, XP002052221
- P. HASTY ET AL.: "The length of homology required for gene targeting in embryonic stem cells." MOLECULAR AND CELLULAR BIOLOGY, Bd. 11, Nr. 11, November 1991, Seiten 5586-5591, XP002052222
- M.R. CAPECCHI ET AL.: "Altering the genome by homologous recombination." SCIENCE, Bd. 244, 16. Juni 1989, Seiten 1288-1292, XP002052223
- R.J. BOLLAG ET AL.: "Homologous recombination in mammalian cells" ANNU. REV. GENET., Bd. 23, 1989, Seiten 199-225, XP002052224
- R.S. KUCHERLAPATI: "Homologous recombination in mammalian somatic cells." PROGRESS IN NUCLEIC ACID RESEARCH AND MOLECULAR BIOLOGY, Bd. 36, 1989, Seiten 301-310, XP002052225
- M.A. VEGA: "Prospects for homologous recombination in human gene therapy." HUMAN GENETICS, Bd. 87, 1991, Seiten 245-253, XP002052226

## Beschreibung

Die Erfindung betrifft ein Verfahren zur Herstellung von Muteinen eukaryontischer Polypeptide in eukaryontischen Zellen mittels homologer Rekombination. Weiterhin betrifft die Erfindung ein Verfahren zur Herstellung humaner Zellen, die zur Herstellung humaner mutierter Proteine geeignet sind. Schließlich betrifft die Erfindung die durch das Verfahren hergestellten humanen Zellen und daraus erhältliche mutierte humane Proteine, sowie pharmazeutische Präparate, die diese Muteine enthalten.

Die Herstellung von rekombinanten humanen Proteinen in großen Mengen im Bereich der Biotechnologie ist bekannt. Auf diese Weise gewonnene Proteine können als therapeutische Mittel verwendet werden. Ebenfalls bekannt ist die rekombinante Herstellung mutierter humaner Proteine, die sich von entsprechenden natürlichen humanen Proteinen durch Deletion, Addition oder/und Substitution einzelner Aminosäuren oder ganzer Peptidabschnitte unterscheiden.

Insbesondere für pharmazeutische Anwendungen ist es oft wünschenswert, humane Polypeptide in eukaryontischen Zellen herzustellen, da diese im Gegensatz zu in prokaryontischen Zellen wie E.coli hergestellten Polypeptiden glykosyliert sind und sich daher von den entsprechenden endogen im Körper vorkommenden Polypeptiden weniger unterscheiden, so daß das Auftreten unerwünschter Nebenwirkungen wie beispielsweise erhöhte Immunogenität oder schlechte Verträglichkeit weniger häufig zu beobachten ist.

Mutierte humane Proteine wurden bisher durch heterologe rekombinante Genexpression hergestellt. Hierzu wurde ein Nukleinsäurekonstrukt in die gewünschte eukaryontische Zelle eingebracht, das die für das mutierte Polypeptid kodierende Nukleinsäuresequenz unter Kontrolle eines Promotors und ein Selektionsmarkergen enthält. Das Nukleinsäurekonstrukt wird bei diesem Vorgang ortsunspezifisch in das Genom der Zelle integriert.

Bei dieser heterologen rekombinanten Genexpression kann es aufgrund der ortsunspezifischen Integration häufig zu unerwünschten und nachteiligen Vorgängen kommen. Beispielsweise kann es während des Integrationsprozesses in das Genom zu Mutationen, insbesondere zu Deletionen in der für das Protein kodierenden Sequenz kommen. Des weiteren kann die Integration an einer Stelle im Genom erfolgen, an der sich Cis-Elemente befinden, die einen reprimierenden Einfluß auf die Expressionskontrollsequenz des Nukleinsäurekonstrukts haben, wodurch Zellen mit verminderter Produktionsleistung für das rekombinante Protein erhalten werden. Eine Integration des Expressionskonstrukts in ein für die Zelle wichtiges Gen führt entweder zum Absterben dieser Zelle oder zu einer rekombinanten Zelle mit Funktionsstörungen, die unter anderem verminderte Ausbeute an rekombinantem Protein zur Folge haben können.

Die Insertion kann aber auch zu einer verminderten Stabilität der so erhaltenen Zellen führen, so daß sie über einen längeren Zeitraum hinweg ihre Fähigkeit zur Expression des rekombinanten Proteins verlieren.

Die der vorliegenden Erfindung zugrundeliegende Aufgabe bestand somit darin, ein Verfahren zur Herstellung von Muteinen eukaryontischer Polypeptide mit einer dem natürlichen Protein möglichst ähnlichen Glykosilierung, in einer stabilen Produktionszelle und in guten Ausbeuten bereitzustellen, um somit die Nachteile des Standes der Technik zumindest teilweise zu beseitigen.

Diese Aufgabe wird erfindungsgemäß gelöst durch ein Verfahren zur Herstellung von Muteinen eukaryontischer Polypeptide,
dadurch gekennzeichnet,
dass man
(i) in eukaryontischen Zellen, die eine für ein endogenes Target-Polypeptid kodierende Target-Nukleinsäuresequenz enthalten, ein zur homologen Rekombination fähiges Nukleinsäuremolekül einbringt, umfassend
   (a) zwei flankierende Sequenzen, die homolog zu Sequenzen im Genlocus der Target-Nukleinsäuresequenz sind, wobei die Flankierenden Sequenzen jeweils eine Länge von mindestens 150 bp haben und Bereiche aus den für das reife Target-Polypeptid kodierenden Sequenzen des Targetgenlocus enthalten, die gegenüber der endogenen Target-Nukleinsäuresequenz eine Mutation im kodierenden Bereich des reifen Target-Polypeptids aufweisen, und
   (b) einen für einen Selektionsmarker kodierenden Nukleinsäureabschnitt,
(ii) die Zellen unter solchen Bedingungen kultiviert, dass eine homologe Rekombination des eingebrachten Nukleinsäuremoleküls stattfindet, wobei die Zelle nach der homologen Rekombination eine mutierte Target-Nukleinsäuresequenz enthält, die zur Expression eines Muteins des Target-Polypeptids in der Lage ist,
(iii) die Zellen, in denen eine homologe Rekombination stattgefunden hat, selektioniert und
(iv) das Mutein aus den Zellen oder/und dem Zellüberstand gewinnt,
wobei zusätzlich die Expression des Targetnukleinsäuresequenz durch Einführen einer heterologen Expressions Kontrollsequenz aktiviert wird.

Durch das erfindungsgemäße Verfahren können mutierte eukaryontische Proteine, insbesondere mutierte humane Proteine in einer homologen Zelle hergestellt werden. Überraschenderweise gelingt es dabei, ein dem natürlichen Protein hinsichtlich des Glykosilierungsmusters möglichst ähnliches mutiertes Protein in hohen Ausbeuten zu gewinnen. Ein Vorteil des erfindungsgemäßen Verfahrens ist, daß ein Protein in einer eukaryontischen Zelle mutiert werden kann und dieses Mutein von dieser Zelle wie das endogen vorkommende Protein der Zelle synthetisiert wird. Ein weiterer Vorteil des erfindungsgemäßen Verfahrens ist, daß die Eigenschaften der resultierenden, das mutierte Protein produzierenden Zellen nicht aufgrund einer ortsunspezifischen Genintegration nachteilig verändert werden. Das Genom der Zelle wird somit außer im Genlocus des zu exprimierenden Proteins in keiner Weise verändert, wodurch die damit verbundenen negativen Effekte ausgeschlossen werden.

Das durch das erfindungsgemäße Verfahren hergestellte humane mutierte Protein unterscheidet sich von dem entsprechenden natürlichen Protein durch Deletion, Addition oder/und Substitution einzelner Aminosäuren oder ganzer Peptidabschnitte. Vorzugsweise werden Muteine hergestellt, die Mutationen am N-Terminus und/oder am C-Terminus aufweisen wie z.B. Deletionen, Insertionen, Substitutionen oder/und Fusionen mit anderen z.B. humanen Proteinen.

Die erfindungsgemäßen Muteine sind vorzugsweise nicht-natürlich vorkommende Polypeptide und unterscheiden sich von natürlich in anderen Ausgangszellen vorkommenden allelischen Variationen des zu mutierenden Polypeptids um mindestens eine Aminosäure. Besonders bevorzugt unterscheiden sich nicht-natürlich vorkommende Muteine durch Deletionen, Additionen oder/und Insertionen einzelner Aminosäuren oder Peptidabschnitte von natürlich vorkommenden allelischen Variationen.

Die im erfindungsgemäßen Verfahren verwendete Zelle ist eine beliebige eukaryontische Zelle, welche mindestens eine endogene Kopie des zu mutierenden Targetgens aufweist. Vorzugsweise ist die Zelle eine humane Zelle, besonders bevorzugt eine immortalisierte humane Zelle wie etwa eine HeLa-Zelle, eine Namalwa-Zelle oder eine HT1080-Zelle.

Überraschenderweise wurde festgestellt, daß bei Verwendung von Ausgangszellen, die eine erhöhte Anzahl von Chromosomen enthalten, auf denen das Targetgen lokalisiert ist, durch homologe Rekombination Zellen erzeugt werden können, die eine erhöhte Ausbeute an mutierten humanen Proteinen erzeugen verglichen mit Zellen, die nur zwei Kopien des Targetgens enthalten. Beispiele für solche Ausgangszellen sind Tumorzellinien mit genetischen Umlagerungen wie etwa HeLaS3 (Puck et al., J. Exp. Med. 103 (1996), 273-284) und Namalwa (Nadkarni et al., Cancer 23 (1969), 64-79), die eine erhöhte Anzahl von Kopien des Chromosom 7 enthalten.

Zur weiteren Verbesserung der Expression des mutierten Polypeptids wird eine endogene Genaktivierung des mutierten Targetgens durchgeführt.

Hierzu werden zusätzliche Sequenzen in das Genom eingebracht, die die Expressionshöhe positiv beeinflussen, wobei z.B. die endogene Expressionskontrollsequenz der Target-Nukleinsäuresequenz mindestens teilweise durch eine heterologe Expressionskontrollsequenz ersetzt wird. Diese heterologe Expressionskontrollsequenz kann einen heterologen Promotor oder/und Enhancer umfassen, vorzugsweise umfasst die heteröloge Expressionskontrollsequenz einen viralen Promotor, insbesondere einen CMV-Promotor. Durch das Ersetzen des endogenen Promotors kann nicht nur die Expression erhöht werden, sondern das Mutein kann bei Verwendung eines geeigneten Promotors synthetisiert werden. Der heterologe Promotor kann ein regulierbarer oder konstruktiver Promotor sein. Außerdem können dadurch auf den endogenen Promotor reprimierend wirkende Cis-Elemente unwirksam gemacht werden. Auch dies kann eine Ausbeutesteigerung bewirken.

Das in die Ausgangszelle eingebrachte Nukleinsäuremolekül umfaßt mindestens einen Sequenzabschnitt, der im Locus des Targetgens eine Integration durch homologe Rekombination erlaubt und dazu geeignet ist, die Mutation im kodierenden Bereich des reifen Targetpolypeptids einzuführen. Das Nukleinsäuremolekül enthalt zwei flankierende Sequenzen, die homolog zu Bereichen des Targetgenlocus sind. Die flankierenden Sequenzen haben jeweils eine Länge von mindestens 150 bp und enthalten Bereiche aus den für das reife Target-Polypeptid kodierenden Sequenzen des Targetgenlocus, die gegenüber der nativen Sequenz modifiziert sind.

Weiterhin enthält das Nukleinsäuremolekül ein Selektionsmarkergen. Dieses kann ein beliebiges für eukaryontische Zellen geeignetes Selektionsmarkergen sein, welches bei Expression zu einem selektierbaren Phänotyp führt, z.B. Antibiotikumresistenz, Auxotrophie, Expression eines Oberflächenproteins etc. Ein besonders bevorzugtes Selektionsmarkergen ist das Neomycinphosphotransferasegen.

Darüber hinaus kann das Nukleinsäuremolekül gegebenenfalls ein negatives Selektionsmarkergen enthalten, z.B. ein HSV-Thymidinkinasegen, durch dessen Expression Zellen in Gegenwart eines selektiven Mittels zerstört werden.

Wenn eine Amplifikation des in der Zelle modifizierten Targetgens gewünscht wird, enthält das Nukleinsäuremolekül ein Amplifikationsgen. Beispiele für geeignete Amplifikationsgene sind Dihydrofolatreduktase, Adenosindeaminase, Ornithindecarboxylase etc. Ein besonders bevorzugtes Amplifikationsgen ist das Dihydrofolatreduktasegen.

Bei Vorhandensein des Amplifikationsgens kann nach der homologen Rekombination eine Amplifikation der mutierten Target-Nukleinsäuresequenz erfolgen, um die Anzahl der Kopien in der Zelle zu erhöhen.

Mit dem erfindungsgemäßen Verfahren können alle im Genom der verwendeten Zelle vorhandenen endogenen Gene mutiert werden. Vorzugsweise ist die Target-Nukleinsäuresequenz eine Gewebsplasminogenaktivator- (tPA), Erythropoietin-, Insulin-, Tumornekrosefaktor-, Interleukin- oder Interleukinrezeptor-Sequenz. Besonders bevorzugt ist das mit dem erfindungsgemäßen Verfahren erhaltene Mutein ein Polypeptid, das sich in seinen biologischen Eigenschaften von dem entsprechenden natürlichen Protein unterscheidet, wie etwa ein von t-PA abgeleitetes Polypeptid, umfassend die K2- und P-Domäne von t-PA (EP 0 382 174).

Zur Gewinnung des Muteins können die hierzu bekannten Techniken verwendet werden. Vorzugsweise erfolgt die Gewinnung des Muteins aus dem Überstand von in Suspension kultivierten Zellen. In Suspension kultivierbare Zellen sind insbesondere für eine Produktion im Großmaßstab von Vorteil. Die im Verlauf des Herstellungsverfahrens nötigen Transfers der kultivierten Zellen werden dadurch erheblich vereinfacht. Dies führt zu einer erheblichen Einsparung an Produktionszeit und Produktionsmitteln und somit zu einer deutlichen Kostenreduzierung. Besonders bevorzugt erfolgt die Gewinnung des Muteins aus dem Überstand von in serumfreiem Medium kultivierten Zellen. Das Mutein kann aus in serumfreiem Medium im Gegensatz zu mit Serum kultiverten Zellen leichter und kostengünstiger isoliert werden, da weniger Reinigungsschritte nötig sind.

Offenbart wird ein mutiertes humanes Polypeptid aus einer humanen Zelle, erhältlich nach einem wie oben beschriebenen Verfahren, gekennzeichnet durch humane Glykosilierung und Abwesenheit speziesfremder Polypeptide. Abwesenheit speziesfremder Polypeptide bedeutet Verunreinigungen speziesfremder Polypeptide von von weniger als 3 Gew.-%, vorzugsweise weniger als 1 Gew.-% und am meisten bevorzugt von weniger als 0,1 Gew.-% bezüglich der Menge an gewünschtem Protein.

Ein weiterer Gegenstand der Erfindung ist ein Verfahren zur Herstellung einer humanen Zelle, die ein Mutein eines humanen Target-Polypeptids exprimiert,
dadurch gekennzeichnet,
dass man
(i) in humane Zellen, die eine für ein endogenes Target-Polypeptid kodierende Target-Nukleinsäuresequenz enthalten, ein Nukleinsäuremolekül einbringt, umfassend
   (a) zwei flankierende Sequenzen, die homolog zu Sequenzen im Genlocus der Target-Nukleinsäuresequenz sind, wobei die Flankierenden Sequenzen jeweils eine Länge von mindestens 150 bp haben und Bereiche aus den für das reife Target-Polypeptid kodierenden Sequenzen des Targetgenlocus enthalten, die gegenüber der endogenen Target-Nukleinsäuresequenz eine Mutation im kodierenden Bereich des reifen Target-Polypeptids aufweisen,
   (b) eine heterologe Expressionskontrollsequenz für die Target-Nukleinsäuresequenz und
   (c) einen für einen Selektionsmarker kodierenden Nukleinsäureabschnitt,
(ii) die Zellen unter solchen Bedingungen kultiviert; dass eine homologe Rekombination des eingebrachten Nukleinsäuremoleküls stattfindet, wobei die Zelle nach der homologen Rekombination eine mutierte Target-Nukleinsäuresequenz enthält, die zur Expression eines Muteins des Target-Polypeptids in der Lage ist,
(iii) die Zellen, in denen eine homologe Rekombination stattgefunden hat, selektioniert und
(iv) die so selektionierten Zellen gewinnt.

In einer bevorzugten Ausführungsform enthält das Nukleinsäuremolekül zusätzlich ein Amplifikationsgen und es erfolgt nach der homologen Rekombination eine Amplifikation der mutierten Target-Nukleinsäuresequenz.

Ein weiterer Gegenstand der Erfindung ist eine humane Zelle, erhältlich durch ein wie oben beschriebenes Verfahren, die mindestens ein für ein mutiertes humanes Polypeptid kodierendes endogenes Gen enthält.

Die erfindungsgemäße Zelle ist unter geeigneten Kulturbedingungen kultivierbar, vorzugsweise ist sie eine in Suspension, besonders bevorzugt eine in serumfreiem Medium wachsende Zelle.

Ein weiterer Gegenstand der Erfindung ist die Verwendung einer humanen Zelle, hergestellt nach einem wie oben beschriebenen Verfahren zur Herstellung eines Muteins eines humanen Polypeptids.

Offenbart wird ein pharmazeutisches Präparat, das dadurch gekennzeichnet ist, daß es ein wie oben beschriebenes Mutein als Wirkstoff gegebenenfalls mit anderen Wirkstoffen oder/und pharmazeutisch üblichen Träger-, Hilfs- oder Zusatzstoffen enthält.

### Beispiel

### Konstruktion einer t-PA Mutante, welche die Domänen K2 und P besitzt:

### a) Vektorkonstruktion

Der Targetingvektor setzt sich aus folgenden Elementen zusammen (aufgelistet in 5'-3' Abfolge):
A: ein 6 kb großes BgIII-Fragment, welches etwa 3,5 kb der 5'-stromaufwärts Region des t-PA Gens enthält (Friezner et al. 1986, JBC 261 (15): 6972)
B: eine etwa 5,2 große Genaktivierungssequenz (als Agel-Fragment), die das Neomycinphosphotransferase-(NEO)-Gen unter Kontrolle des RSV-Promotors und der späten Polyadenylierungsstelle von SV40 als Terminator, ein für eine Arginin-Mutante der murinen Dihydrofolatreduktase (DHFR) kodierendes Gen (Simonsen et al., Proc. Natl. Acad. Sci. USA 80 (1983), 2495) unter Kontrolle des frühen SV40 Promotors und der frühen SV40 Polyadenylierungsstelle als Terminator (Kaufmann et al., Mol. Cell. Biol. 2 (1982), 1304; Okayama et al., Mol. Cell. Biol. 3 (1983), 280 und Schimke, J. Biol. Chem. 263 (1988), 5989) und den Cytomegalovirus (CMV)-Promoter (Boshart et al., Cell 41 (1995), S 21) enthält
C: ein etwa 200 bp großes Fragment, isoliert aus der t-PA cDNA, das den Nukleotidpositionen 1-199 entspricht und das die kodierende Region für die Signalsequenz und den ersten drei Aminosäuren des maturen t-PA enthält (Pennica et al. 1983, Nature 301:214)
D: ein etwa 1,5 kb großes EcoRI-Fragment, das einen großen Teil des Intron G des tPA-Gens umfaßt (Friezner et al. op. cit., Ny et al. 1984, PNAS 81:5355).

Diese Elemente wurden aus den entsprechenden Ausgangsmaterialien via PCR und geeigneten Fusions-PCR-Primern isoliert und zusammengefügt. Anschließend wurden die fusionierten Elemente in pBR322 ligiert und in E.coli eingebracht. Alternativ können die Fragmente auch aus den jeweiligen Ausgangsmaterialien herausgeschnitten und über Linker ligiert werden.

### b) Humane Zellinie

Als Zellinie zur Durchführung der endogenen Genaktivierung wurde HeLa verwendet, von der gezeigt werden konnte, daß die Transkription des t-PA Gens durch Zugabe von Phorbolmyristatacetat induziert werden kann (Waller und Schleuning 1985, J. Biol. Chem. 260:6354). Nach Einbringung des Targetingvektors via Elektroporation wurden die Vektor enthaltenden Zellen durch Zugabe von G418 selektioniert. Die Zellen, die aufgrund einer homologen Rekombination ein Polypeptid mit den t-PA-Domänen K2 und P sezernieren, wurden aufgefunden, indem man den Überstand der Zellen mit einem ELISA (Imubind-Total t-PA, American Diagnostics) testete, der in der Lage ist, die Expression des gesuchten Polypeptids nachzuweisen.

## Patentansprüche

1. Verfahren zur Herstellung von Muteinen eukaryontischer Polypeptide,
**dadurch gekennzeichnet,**
**dass** man
(i) in eukaryontischen Zellen, die eine für ein endogenes Target-Polypeptid kodierende Target-Nukleinsäuresequenz enthalten, ein zur homologen Rekombination fähiges Nukleinsäuremolekül einbringt, umfassend
(a) zwei flankierende Sequenzen, die homolog zu Sequenzen im Genlocus der Target-Nukleinsäuresequenz sind, wobei die Flankierenden Sequenzen jeweils eine Länge von mindestens 150 bp haben, und Bereiche aus den für das reife Target-Polypeptid kodierenden Sequenzen des Targetgenlocus enthalten, die gegenüber der endogenen Target-Nukleinsäuresequenz eine Mutation im kodierenden Bereich des reifen Target-Polypeptids aufweisen, und
(b) einen für einen Selektionsmarker kodierenden Nukleinsäureabschnitt,
(ii) die Zellen unter solchen Bedingungen kultiviert, dass eine homologe Rekombination des eingebrachten Nukleinsäuremoleküls stattfindet, wobei die Zelle nach der homologen Rekombination eine mutierte Target-Nukleinsäuresequenz enthält, die zur Expression eines Muteins des Target-Polypeptids in der Lage ist,
(iii) die Zellen, in denen eine homologe Rekombination stattgefunden hat, selektioniert und
(iv) das Mutein aus den Zellen oder/und dem Zellüberstand gewinnt,
wobei zusätzlich die Expression des Tangetnukleinsäuresequenz durch Einführen einer heterologen Expressions kontrollsequenz aktiviert wird.

2. Verfahren nach Anspruch 1,
**dadurch gekennzeichnet,**
**dass** die Zelle eine humane Zelle ist.

3. Verfahren nach Anspruch 2,
**dadurch gekennzeichnet,**
**dass** die Zelle eine HeLa-Zelle, eine Namalwa-Zelle oder eine HT1080 Zelle ist.

4. Verfahren nach einem der Ansprüche 1 bis 3,
**dadurch gekennzeichnet,**
**dass** man eine Ausgangszelle verwendet, die die Target-Nukleinsäuresequenz auf multiplen Chromosomen enthält.

5. Verfahren nach Anspruch 1,
**dadurch gekennzeichnet,**
**dass** die heterologe Expressionskontrollsequenz ein viraler Promotor, insbesondere ein CMV-Promotor ist.

6. Verfahren nach einem der vorhergehenden Ansprüche,
**dadurch gekennzeichnet,**
**dass** der Selektionsmarker-kodierende Nukleinsäureabschnitt ein Neomycinphosphotransferasegen ist.

7. Verfahren nach einem der vorhergehenden Ansprüche,
**dadurch gekennzeichnet,**
**dass** das in die Zelle eingebrachte Nukleinsäuremolekül zusätzlich ein Amplifikationsgen enthält und nach der homologen Rekombination eine Amplifikation der mutierten Target-Nukleinsäuresequenz erfolgt.

8. Verfahren nach Anspruch 7,
**dadurch gekennzeichnet,**
**dass** man ein Dihydrofolatreduktasegen als Amplifikationsgen verwendet.

9. Verfahren nach einem der vorhergehenden Ansprüche,
**dadurch gekennzeichnet,**
**dass** die Target-Nukleinsäuresequenz eine Gewebsplasminogen aktivator- (t-PA), Erythropoietin-, Insulin-, Tumornekrosefaktor-, Interleukin- oder Interleukinrezeptor-Sequenz ist.

10. Verfahren nach einem der Ansprüche 1 bis 8,
**dadurch gekennzeichnet,**
**dass** das Mutein ein von t-PA abgeleitetes Polypeptid ist, umfassend die K2- und P-Domäne von t-PA.

11. Verfahren nach einem der vorhergehenden Ansprüche,
**dadurch gekennzeichnet,**
**dass** die Gewinnung des Muteins aus dem Überstand von in Suspension kultivierten Zellen erfolgt.

12. Verfahren nach einem der vorhergehenden Ansprüche,
**dadurch gekennzeichnet,**
**dass** die Gewinnung des Muteins aus dem Überstand von in serumfreiem Medium kultivierten Zellen erfolgt.

13. Verfahren zur Herstellung einer humanen Zelle, die ein Mutein eines humanen Target-Polypeptids exprimiert,
**dadurch gekennzeichnet,**
**dass** man
(i) in humane Zellen, die eine für ein endogenes Target-Polypeptid kodierende Target-Nukleinsäuresequenz enthalten, ein Nukleinsäuremolekül einbringt, umfassend
(a) zwei flankierende Sequenzen, die homolog zu Sequenzen im Genlocus der Target-Nukleinsäuresequenz sind, wobei die Flankierenden Sequenzen jeweils eine Länge von mindestens 150 bp haben und Bereiche aus den für das reife Target-Polypeptid kodierenden Sequenzen des Targetgenlocus enthalten, die gegenüber der endogenen Target-Nukleinsäuresequenz eine Mutation im kodierenden Bereich des reifen Target-Polypeptids aufweisen,
(b) eine heterologe Expressionskontrollsequenz für die Target-Nukleinsäuresequenz und
(c) einen für einen Selektionsmarker kodierenden Nukleinsäureabschnitt,
(ii) die Zellen unter solchen Bedingungen kultiviert, dass eine homologe Rekombination des eingebrachten Nukleinsäuremoleküls stattfindet, wobei die Zelle nach der homologen Rekombination eine mutierte Target-Nukleinsäuresequenz enthält, die zur Expression eines Muteins des Target-Polypeptids in der Lage ist,
(iii) die Zellen, in denen eine homologe Rekombination stattgefunden hat, selektioniert und
(iv) die so selektionierten Zellen gewinnt.

14. Verfahren nach Anspruch 13,
**dadurch gekennzeichnet,**
**dass** das Nukleinsäuremolekül zusätzlich ein Amplifikationsgen enthält und nach der homologen Rekombination eine Amplifikation der mutierten Target-Nukleinsäuresequenz erfolgt.

15. Verfahren nach Anspruch 14,
**dadurch gekennzeichnet,**
**dass** man ein Dihydrofolatreduktasegen als Amplifikationsgen verwendet.

16. Humane Zelle, erhältlich durch ein Verfahren nach einem der Ansprüche 13 bis 15, die mindestens ein für ein mutiertes humanes Polypeptid kodierendes endogenes Gen enthält, wobei die Zelle **dadurch** erhältlich ist, dass man
(i) in humane Zellen, die eine für ein endogenes Target-Polypeptid kodierende Target-Nukleinsäuresequenz enthalten, ein Nukleinsäuremolekül einbringt, umfassend
(a) zwei flankierende Sequenzen, die homolog zu Sequenzen im Genlocus der Target-Nukleinsäuresequenz sind, wobei die Flankierenden Sequenzen jeweils eine Länge von mindestens 150 bp haben und Bereiche aus den für das reife Target-Polypeptid kodierenden Sequenzen des Targetgenlocus enthalten, die
gegenüber der endogenen Target-Nukleinsäuresequenz eine Mutation im kodierenden Bereich des reifen Target-Polypeptids aufweisen,
(b) eine heterologe Expressionskontrolisequenz für die Target-Nukleinsäuresequenz und
(c) einen für einen Selektionsmarker kodierenden Nukleinsäureabschnitt,
(ii) die Zellen unter solchen Bedingungen kultiviert, dass eine homologe Rekombination des eingebrachten Nukleinsäuremoleküls stattfindet, wobei die Zelle nach der homologen Rekombination eine mutierte Target-Nukleinsäuresequenz enthält, die zur Expression eines Muteins des Target-Polypeptids in der Lage ist,
(iii) die Zellen, in denen eine homologe Rekombination stattgefunden hat, selektioniert und
(iv) die so selektionierten Zellen gewinnt.

17. Verwendung einer humanen Zelle nach Anspruch 16 zur Herstellung eines Muteins eines humanen Polypeptids.

18. Verwendung eines Muteins nach Anspruch 17 zur Herstellung eines pharmazeutischen Präparates,
**dadurch gekennzeichnet,**
**dass** es das Mutein als Wirkstoff gegebenenfalls mit anderen Wirkstoffen oder/und pharmazeutisch üblichen Träger-, Hilfs- oder Zusatzstoffen enthält.

## Claims

1. Process for the production of muteins of eukaryotic polypeptides **characterized in that**
(i) a nucleic acid molecule capable of homologous recombination is introduced into eukaryotic cells which contain a target nucleic acid sequence coding for an endogenous target polypeptide, the said nucleic acid molecule comprising
(a) two flanking sequences which are homologous to sequences in the gene locus of the target nucleic acid sequence, wherein the flanking sequences each have a length of at least 150 bp and contain regions from the sequences of the target gene locus coding for the mature target polypeptide which, compared to the endogenous target nucleic acid sequence have a mutation in the coding region of the mature target polypeptide and
(b) a nucleic acid section coding for a selection marker,
(ii) the cells are cultured under such conditions that a homologous recombination of the introduced nucleic acid molecule takes place whereby the cell contains a mutated target nucleic acid sequence after the homologous recombination which is able to express a mutein of the target polypeptide,
(iii) the cells, in which a homologous recombination has taken place, are selected and
(iv) the mutein is isolated from the cells or/and the cell supernatant, wherein in addition the expression of the target nucleic acid sequence is activated by inserting a heterologous expression control sequence.

2. Process according to claim 1,
**characterized in that**
the cell is a human cell.

3. Process according to claim 2,
**characterized in that**
the cell is a HeLa cell, a Namalwa cell or a HT1080 cell.

4. Process according to one of the claims 1 to 3,
**characterized in that**
a starting cell is used which contains the target nucleic acid sequence on multiple chromosomes.

5. Process according to claim 1,
**characterized in that**
the heterologous expression control sequence is a viral promoter and in particular a CMV promoter.

6. Process according to one of the previous claims,
**characterized in that**
the nucleic acid section coding for the selection marker is a neomycin phosphotransferase gene.

7. Process according to one of the previous claims,
**characterized in that**
the nucleic acid molecule introduced into the cell additionally contains an amplification gene and the mutated target nucleic acid sequence is amplified after the homologous recombination.

8. Process according to claim 7,
**characterized in that**
a dihydrofolate reductase gene is used as an amplification gene.

9. Process according to one of the previous claims,
**characterized in that**
the target nucleic acid sequence is a tissue plasminogen activator (t-PA), erythropoietin, insulin, tumour necrosis factor, interleukin or interleukin receptor sequence.

10. Process according to one of the claims 1 to 8,
**characterized in that**
the mutein is a polypeptide derived from t-PA comprising the K2 and P domain of t-PA.

11. Process according to one of the previous claims,
**characterized in that**
the mutein is isolated from the supernatant of cells cultured in suspension.

12. Process according to one of the previous claims,
**characterized in that**
the mutein is isolated from the supernatant of cells cultured in serum-free medium.

13. Process for the production of a human cell which expresses a mutein of a human target polypeptide,
**characterized in that**
(i) a nucleic acid molecule is introduced into human cells which contain a target nucleic acid sequence coding for an endogenous target polypeptide, the said nucleic acid molecule comprising
(a) two flanking sequences which are homologous to sequences in the gene locus of the target nucleic acid sequence, wherein the flanking sequences each have a length of at least 150 bp and contain regions from the sequences of the target gene locus coding for the mature target polypeptide which, compared to the endogenous target nucleic acid sequence have a mutation in the coding region of the mature target polypeptide,
(b) a heterologous expression control sequence for the target nucleic acid sequence and
(c) a nucleic acid section coding for a selection marker,
(ii) the cells are cultured under such conditions that a homologous recombination of the introduced nucleic acid molecule takes place whereby the cell contains a mutated target nucleic acid sequence after the homologous recombination which is able to express a mutein of the target polypeptide,
(iii) the cells, in which a homologous recombination has taken place, are selected and
(iv) the cells selected in this manner are isolated.

14. Process according to claim 13,
**characterized in that**
the nucleic acid molecule additionally contains an amplification gene and, after the homologous recombination, the mutated target nucleic acid sequence is amplified.

15. Process according to claim 14,
**characterized in that**
a dihydrofolate reductase gene is used as an amplification gene.

16. Human cell obtainable by a process according to one of the claims 13 to 15 which contains at least one endogenous gene which codes for a mutated human polypeptide, wherein the cell can be obtained by
(i) introducing a nucleic acid molecule into human cells which contain a target nucleic acid sequence coding for an endogenous target polypeptide, the said nucleic acid molecule comprising
(a) two flanking sequences which are homologous to sequences in the gene locus of the target nucleic acid sequence, wherein the flanking sequences each have a length of at least 150 bp and contain regions from the sequences of the target gene locus coding for the mature target polypeptide which, compared to the endogenous target nucleic acid sequence have a mutation in the coding region of the mature target polypeptide,
(b) a heterologous expression control sequence for the target nucleic acid sequence and
(c) a nucleic acid section coding for a selection marker,
(ii) culturing the cells under such conditions that a homologous recombination of the introduced nucleic acid molecule takes place whereby the cell contains a mutated target nucleic acid sequence after the homologous recombination which is able to express a mutein of the target polypeptide,
(iii) selecting the cells in which a homologous recombination has taken place and
(iv) isolating the cells selected in this manner.

17. Use of a human cell according to claim 16 for the production of a mutein of a human polypeptide.

18. Use of a mutein according to claim 17 to produce a pharmaceutical preparation,
**characterized in that**
it contains the mutein as an active substance optionally together with other active substances or/and common pharmaceutical carriers, auxiliary substances or additives.

## Revendications

1. Procédé de préparation de mutéines de polypeptides eucaryotes, **caractérisé en ce que**
(i) dans des cellules eucaryotes qui contiennent une séquence d'acide nucléique cible codant pour un polypeptide cible endogène, on introduit une molécule d'acide nucléique apte à une recombinaison homologue, comprenant:
(a) deux séquences flanquantes qui sont homologues à des séquences dans le locus du gène de la séquence d'acide nucléique cible, les séquences flanquantes ayant chacune une longueur d'au moins 150 pb, et contiennent des régions provenant des séquences du locus du gène cible codant pour le polypeptide cible mature qui présentent, par rapport à la séquence d'acide nucléique cible endogène, une mutation dans la région codante du polypeptide cible mature, et
(b) un fragment d'acide nucléique codant pour un marqueur de sélection,
(ii) on cultive les cellules dans des conditions telles qu'il se produise une recombinaison homologue de la molécule d'acide nucléique introduite, grâce à quoi la cellule contient après la recombinaison homologue une séquence d'acide nucléique cible mutée qui est capable d'exprimer une mutéine du polypeptide cible,
(iii) on sélectionne les cellules dans lesquelles s'est produite une recombinaison homologue, et
(iv) on récupère la mutéine à partir des cellules et/ou du surnageant des cellules, l'expression de la séquence d'acide nucléique cible étant activée par l'introduction d'une séquence de contrôle de l'expression hétérologue.

2. Procédé selon la revendication 1, **caractérisé en ce que** la cellule est une cellule humaine.

3. Procédé selon la revendication 2, **caractérisé en ce que** la cellule est une cellule HeLa, une cellule Namalwa ou une cellule HT1080.

4. Procédé selon l'une des revendications 1 à 3, **caractérisé en ce que** l'on utilise une cellule de départ qui contient la séquence d'acide nucléique cible sur de multiples chromosomes.

5. Procédé selon la revendication 1, **caractérisé en ce que** la séquence de contrôle de l'expression hétérologue est un promoteur viral, en particulier un promoteur de CMV.

6. Procédé selon l'une des revendications précédentes, **caractérisé en ce que** le fragment d'acide nucléique codant pour un marqueur de sélection est un gène de néomycine-phosphotransférase.

7. Procédé selon l'une des revendications précédentes, **caractérisé en ce que** la molécule d'acide nucléique introduite dans la cellule contient en outre un gène d'amplification et, après la recombinaison homologue, il se produit une amplification de la séquence d'acide nucléique cible mutée.

8. Procédé selon la revendication 7, **caractérisé en ce que** l'on utilise un gène de dihydrofolate-réductase comme gène d'amplification.

9. Procédé selon l'une des revendications précédentes, **caractérisé en ce que** la séquence d'acide nucléique cible est une séquence de l'activateur tissulaire du plasminogène (t-PA), de l'érythropoïétine, de l'insuline, du facteur de nécrose tumorale, d'une interleukine ou d'un récepteur d'interleukine.

10. Procédé selon l'une des revendications 1 à 8, **caractérisé en ce que** la mutéine est un polypeptide dérivé du t-PA, comprenant les domaines K2 et P du t-PA.

11. Procédé selon l'une des revendications précédentes, **caractérisé en ce que** la récupération de la mutéine s'effectue à partir du surnageant de cellules cultivées en suspension.

12. Procédé selon l'une des revendications précédentes, **caractérisé en ce que** la récupération de la mutéine s'effectue à partir du surnageant de cellules cultivées dans un milieu sans sérum.

13. Procédé de préparation d'une cellule humaine qui exprime une mutéine d'un polypeptide cible humain, **caractérisé en ce que**
(i) dans des cellules humaines qui contiennent une séquence d'acide nucléique cible codant pour un polypeptide cible endogène, on introduit une molécule d'acide nucléique comprenant:
(a) deux séquences flanquantes qui sont homologues à des séquences dans le locus du gène de la séquence d'acide nucléique cible, les séquences flanquantes ayant chacune une longueur d'au moins 150 pb, et contiennent des régions provenant des séquences du locus du gène cible codant pour le polypeptide cible mature qui présentent, par rapport à la séquence d'acide nucléique cible endogène, une mutation dans la région codante du polypeptide cible mature,
(b) une séquence de contrôle de l'expression hétérologue pour la séquence d'acide nucléique cible, et
(c) un fragment d'acide nucléique codant pour un marqueur de sélection,
(ii) on cultive les cellules dans des conditions telles qu'il se produise une recombinaison homologue de la molécule d'acide nucléique introduite, grâce à quoi la cellule contient après la recombinaison homologue une séquence d'acide nucléique cible mutée qui est capable d'exprimer une mutéine du polypeptide cible,
(iii) on sélectionne les cellules dans lesquelles s'est produite une recombinaison homologue, et
(iv) on récupère les cellules ainsi sélectionnées.

14. Procédé selon la revendication 13, **caractérisé en ce que** la molécule d'acide nucléique contient en outre un gène d'amplification et, après la recombinaison homologue, il se produit une amplification de la séquence d'acide nucléique cible mutée.

15. Procédé selon la revendication 14, **caractérisé en ce que** l'on utilise un gène de dihydrofolate-réductase comme gène d'amplification.

16. Cellule humaine, pouvant être obtenue par un procédé selon l'une des revendications 13 à 15, qui contient au moins un gène endogène codant pour un polypeptide humain muté, la cellule pouvant être obtenue par un procédé selon lequel
(i) dans des cellules humaines qui contiennent une séquence d'acide nucléique cible codant pour un polypeptide cible endogène, on introduit une molécule d'acide nucléique comprenant:
(a) deux séquences flanquantes qui sont homologues à des séquences dans le locus du gène de la séquence d'acide nucléique cible, les séquences flanquantes ayant chacune une longueur d'au moins 150 pb, et contiennent des régions provenant des séquences du locus du gène cible codant pour le polypeptide cible mature qui présentent, par rapport à la séquence d'acide nucléique cible endogène, une mutation dans la région codante du polypeptide cible mature,
(b) une séquence de contrôle de l'expression hétérologue pour la séquence d'acide nucléique cible, et
(c) un fragment d'acide nucléique codant pour un marqueur de sélection,
(ii) on cultive les cellules dans des conditions telles qu'il se produise une recombinaison homologue de la molécule d'acide nucléique introduite, grâce à quoi la cellule contient après la recombinaison homologue une séquence d'acide nucléique cible mutée qui est capable d'exprimer une mutéine du polypeptide cible,
(iii) on sélectionne les cellules dans lesquelles s'est produite une recombinaison homologue, et
(iv) on récupère les cellules ainsi sélectionnées.

17. Utilisation d'une cellule humaine selon la revendication 16 pour la préparation d'une mutéine d'un polypeptide humain.

18. Utilisation d'une mutéine selon la revendication 17 pour la préparation d'une composition pharmaceutique **caractérisée en ce qu'**elle contient la mutéine comme substance active, éventuellement avec d'autres substances actives et/ou des supports, des agents auxiliaires ou des additifs classiques en pharmacie.
